# EUROPEAN PATENT APPLICATION

(11) **EP 2 373 045 A1**
(43) Date of publication of application: **05.10.2011**
(21) Application number: 11152581.2
(22) Date of filing: 28.01.2011
(51) Int. Cl.: H04N 13/00

(54) **Medical image generating apparatus, medical image display apparatus, medical image generating method and program**

(30) Priority: 04.03.2010 JP 2010047758
(71) Applicant: Fujifilm Corporation, Minato-ku Tokyo 106-8620 (JP)
(72) Inventor: Kusunoki, Tetsuro, Kanagawa 258-8538 (JP)
(74) Representative: Klunker, Hans-Friedrich

(57) **Abstract**

3D image areas (144r, 1441) are set respectively from within display areas (140r, 1401) of a right-eye image display unit (36) and a left-eye image display unit (34), and 2D image areas (146r, 1461) are set respectively from within the 3D image areas (144r, 1441). A right-eye disparity image (42r) and a left-eye disparity image (421) to be displayed respectively in the 3D image areas (144r, 1441) are acquired, and planar images 74 to be displayed respectively in the 2D image areas (146r, 1461) are acquired. The planar images (74) are placed respectively in the 2D image areas (146r, 1461), whereas the right-eye disparity image (42r) and the left-eye disparity image (421) are placed in the 3D image areas (144r, 1441) apart from the 2D image areas (146r, 1461), thereby generating a right-eye image (78r) and a left-eye image (781).

## Description

### BACKGROUND OF THE INVENTION

### Field of the Invention:

The present invention relates to a medical image generating apparatus, a medical image display apparatus, a medical image generating method, and a program for generating a pair of two-dimensional medical images to be displayed respectively on a pair of display units, which provide a three-dimensional image display based on binocular disparity.

### Description of the Related Art:

Basically, image data acquired by medical diagnosing apparatus, such as X-ray diagnosing apparatus, MRI diagnosing apparatus, various CT apparatus, etc., are output and visualized in two different ways, i.e., as a hard copy on a medium such as a light-permeable X-ray film carrying a captured image recorded thereon, and as a soft copy, which is made up of image information displayed on a display unit such as a CRT, an LCD, or the like (hereinafter simply referred to as a "display"). In recent years, more and more diagnostic images are viewed on displays, rather than being recorded on X-ray films or the like, for many reasons including demands for cost reduction, better image management, and advances in communication technology.

A two-dimensional radiographic image represents a projected image of a radiation dose that is transmitted through (and absorbed by) a subject, i.e., a grayscale image acquired of various regions of a subject, which are positioned in a superposed relationship along a direction in which radiation is applied to the subject. The doctor or radiological technician in charge of the subject sees and interprets the acquired two-dimensional radiographic image in order to develop an accurate grasp of a three-dimensional positional relationship between regions of the subject, as well as the presence and shape of possible small lesions in the subject. Therefore, the doctor or radiological technician is required to be highly skilled and must be quite careful in interpreting two-dimensional radiographic images.

If radiographic images can be observed as a three-dimensional image based on binocular disparity, then it is expected that the ability to diagnose radiographic images will be greatly improved, because the observer is able to grasp depth information from the radiographic image much more easily than from a planar or two-dimensional radiographic image. Three-dimensional radiographic images can only be output as soft copies, which allows image data to be regenerated and reprocessed at any time and then immediately updated, without the need for expendable mediums such as hard-copy mediums.

As with general medical apparatus, displays for use in diagnostic applications are required to meet demands for both image diagnosis and display controllability. For displaying three-dimensional images, the displays need to cope with problems unique to three-dimensional images, unlike ordinary two-dimensional images. In order to solve such problems, there have been proposed various image display technologies in various fields of application, as described below.

Japanese Laid-Open Patent Publication No. 2002-091643 discloses an apparatus for and a method of controlling three-dimensional image displays by connecting 2D (two-dimensional) displays to direct-view-type 3D (three-dimensional) displays. The publication states that one disadvantage of direct-view-type 3D displays, i.e., lack of high resolution, can be corrected by displaying a GUI such as icons or the like on the 2D displays.

Japanese Laid-Open Patent Publication No. 2009-018184 discloses a method of and a system for medical three-dimensional image capture and navigation. According to the disclosed method and system, a three-dimensional dynamic image of an organ to be examined is displayed. More specifically, a reconstructed three-dimensional image of the organ is displayed in one window, and a real-time two-dimensional image of the heart is displayed in another window next to the one window (see paragraphs [0206] through [0208], FIGS. 16B, 16C, etc., of the publication). The publication mentions that diagnostic information, including a three-dimensional image, can be displayed as various images.

Japanese Laid-Open Patent Publication No. 2005-175538 discloses an apparatus for and a method of displaying a two-dimensional image in all selected image areas or in an image area of a display area of a direct-view-type 3D display, and for displaying a three-dimensional image in a remaining image area. According to this publication, it is stated that the disclosed apparatus can be used in a wider range, whereby the apparatus is made more practical.

For performing an image diagnosis on displays, it is customary to use two diagnostic monochromatic displays and one multipurpose color display in combination. In order to achieve an image rendering capability equivalent to X-ray films, the monochromatic displays, e.g., LCDs, incorporate a display panel having a higher resolution (smaller pixel size) and a backlight exhibiting higher brightness than general-purpose displays.

It has been the practice in the art of medical imaging for the doctor or radiological technician to discover an abnormality in a mass to be examined by displaying a portion of a two-dimensional image of the mass at an enlarged scale on a display screen, and scrutinizing the enlarged image that is displayed on the display screen.

According to research conducted by the inventor of the present invention, it has been found that the ability to diagnose images is greatly increased by interpreting stereographic images (three-dimensional images) and planar images (two-dimensional images) in appropriate combinations thereof.

The apparatus disclosed in Japanese Laid-Open Patent Publication No. 2002-091643, however, uses four displays, i.e., two direct-view-type 3D displays and two 2D displays, rather than two diagnostic monochromatic displays. The large number of displays involved tends to be detrimental to the process of interpreting displayed images, as it is quite burdensome for an observer to pay attention to all of the displayed images.

The system disclosed in Japanese Laid-Open Patent Publication No. 2009-018184 poses limitations on image areas that can be displayed on a single display, because the display has a three-dimensional image display window and a two-dimensional image display window, which are positioned proximate to each other.

The apparatus disclosed in Japanese Laid-Open Patent Publication No. 2005-175538 needs a large hardware configuration and hence is costly to manufacture, because it handles a large amount of data for signal processing. In particular, it is quite difficult for the disclosed apparatus to enable increased image diagnosis for mammographic processes, which display high-definition images for image interpretation.

### SUMMARY OF THE INVENTION

An object of the present invention is to provide a medical image generating apparatus, a medical image display apparatus, a medical image generating method, and a program, which are effective at greatly increasing the ability to diagnose images with respect to a three-dimensional image display based on binocular disparity.

According to the present invention, there is provided a medical image generating apparatus for generating a pair of two-dimensional images to be displayed respectively on a pair of display units for providing a stereographic image display based on binocular disparity.

The medical image generating apparatus comprises a first image area setting unit for setting image areas for stereographic image display respectively from within display areas of the respective display units, a second image area setting unit for setting image areas for planar image display from within the image areas for stereographic image display, set by the first image area setting unit, a first image acquiring unit for acquiring a pair of disparity images to be displayed respectively in the image areas for stereographic image display, set by the first image area setting unit, a second image acquiring unit for acquiring one image to be displayed in the image areas for planar image display, set by the second image area setting unit, and an image placing unit for placing the one image acquired by the second image acquiring unit in the image areas for planar image display, and placing the disparity images acquired by the first image acquiring unit in the image areas for stereographic image display, apart from the image areas for planar image display, thereby generating the pair of two-dimensional images.

Since the medical image generating apparatus includes the second image area setting unit for setting image areas for planar image display from within the image areas set for stereographic image display, and the image placing unit for placing a pair of disparity images in the image areas for stereographic image display apart from the image areas for planar image display, planar images are placed over a stereographic image for enabling visual observation thereof, and hence the display space required for such image areas is reduced. Since the amount of diagnostic image information that can be observed at a time is increased, the ability to diagnose images is greatly increased.

Preferably, the medical image generating apparatus further includes a frame image adder for adding an image of a light blocking frame, near a boundary between the image areas for stereographic image display and the image areas for planar image display. The light blocking frame is effective to reduce glare from the planar images when the planar images are observed, so that an observer can observe the planar images easily. Since the light blocking frame clearly separates the stereographic image from the planar image at a boundary thereof, the observer finds it easy to observe the stereographic image and the planar image independently of each other.

Preferably, the medical image generating apparatus further includes a first identifying image adder for adding an image of an identification mark for identifying either one of the disparity images, one image of which has been acquired by the second image acquiring unit.

Preferably, the medical image generating apparatus further includes an image area extracting unit for extracting a prescribed image area from within the image areas for stereographic image display. The second image acquiring unit acquires, as the one image, a planar image corresponding to the position of the prescribed image area extracted by the image area extracting unit. When the stereographic image is observed, the stereographic image can be observed in detail in combination with the planar image with respect to an image area of interest.

Preferably, the medical image generating apparatus further includes an image scaling processor for scaling the planar image to match a size of the prescribed image area to a size of the image areas for planar image display. Thus, the planar image can be displayed at a certain size, which depends on the size of the image area that has been set.

Preferably, the image scaling processor further scales the planar image displayed in the image areas for planar image display, depending on an image magnification ratio that has been set.

Preferably, the medical image generating apparatus further includes an identifying image adder for adding an image of an identification mark, which defines the prescribed image area. The relationship between the displayed positions of the stereographic image and the planar image is thus visualized to enable the observer to recognize the relationship easily.

Preferably, the pair of disparity images comprises a pair of radiographic images acquired by a radiation detector, which detects radiation that has passed through a subject, when a radiation source positioned at respective different angles with respect to the radiation detector applies radiation through the subject to the radiation detector.

According to the present invention, there also is provided a medical image display apparatus comprising a pair of display units, a medical image generating apparatus for generating a pair of two-dimensional images to be displayed respectively on the display units for providing a stereographic image display based on binocular disparity, and an optical adjuster for adjusting optical paths and polarized states of light images from the display units for thereby enabling an observer to visually recognize the light images as a stereographic representation. The medical image generating apparatus comprises a first image area setting unit for setting image areas for stereographic image display respectively from within display areas of the respective display units, a second image area setting unit for setting image areas for planar image display from within the image areas for stereographic image display, set by the first image area setting unit, a first image acquiring unit for acquiring a pair of disparity images to be displayed respectively in the image areas for stereographic image display, set by the first image area setting unit, a second image acquiring unit for acquiring one image to be displayed in the image areas for planar image display, set by the second image area setting unit, and an image placing unit for placing the one image acquired by the second image acquiring unit in the image areas for planar image display, and placing the disparity images acquired by the first image acquiring unit in the image areas for stereographic image display, apart from the image areas for planar image display, thereby generating the pair of two-dimensional images.

According to the present invention, there is further provided a medical image generating method for generating a pair of two-dimensional images to be displayed respectively on a pair of display units for providing a stereographic image display based on binocular disparity, comprising the steps of setting image areas for stereographic image display respectively from within display areas of the respective display units, setting image areas for planar image display from within the image areas for stereographic image display, which have been set, acquiring a pair of disparity images to be displayed respectively in the image areas for stereographic image display, which have been set, acquiring one image to be displayed in the image areas for planar image display, which have been set, and placing the one image that has been acquired, in the image areas for planar image display, and placing the disparity images that have been acquired, in the image areas for stereographic image display, apart from the image areas for planar image display, thereby generating the pair of two-dimensional images.

According to the present invention, there is also provided a recording medium storing a program to be executed by a computer on a pair of disparity images to be displayed for providing a stereographic image display based on binocular disparity. The program enables the computer to function as a first image area setting unit for setting image areas for stereographic image display respectively from within display areas of a pair of respective display units, a second image area setting unit for setting image areas for planar image display from within the image areas for stereographic image display, set by the first image area setting unit, a first image acquiring unit for acquiring the pair of disparity images to be displayed respectively in the image areas for stereographic image display, set by the first image area setting unit, a second image acquiring unit for acquiring one image to be displayed in the image areas for planar image display, set by the second image area setting unit, and an image placing unit for placing the one image acquired by the second image acquiring unit in the image areas for planar image display, and placing the disparity images acquired by the first image acquiring unit in the image areas for stereographic image display, apart from the image areas for planar image display, thereby generating the pair of two-dimensional images.

With the medical image generating apparatus, the medical image display apparatus, the medical image generating method, and the program according to the present invention, image areas for stereographic image display are set respectively from within the display areas of respective display units, and image areas for planar image display are set respectively from within the image areas for stereographic image display. A pair of disparity images, which are to be displayed in the set image areas for stereographic image display, and one image, which is to be displayed in the set image areas for planar image display, are acquired. The acquired one image is set in the image areas for planar image display, and the disparity images acquired by the first image acquiring unit are placed in the image areas for stereographic image display, apart from the image areas for planar image display, thereby generating the pair of two-dimensional images. Consequently, planar images are placed over a stereographic image for visual observation, and hence the display space required for the image areas is reduced. Since the amount of information in the diagnostic images that can be observed at a time is increased, the image diagnostic ability is greatly increased.

The above and other objects, features, and advantages of the present invention will become more apparent from the following description when taken in conjunction with the accompanying drawings in which a preferred embodiment of the present invention is shown by way of illustrative example.

### BRIEF DESCRIPTION OF THE DRAWINGS

FIG. 1 is a schematic diagram of a radiographic image capturing system incorporating a medical image display apparatus according to an embodiment of the present invention;
FIG. 2A is a view showing a right-eye image as a two-dimensional image;
FIG. 2B is a view showing a left-eye image as a two-dimensional image;
FIG. 2C is a view showing a stereographic image (three-dimensional image);
FIG. 3 is a perspective view of a mammographic apparatus, which serves as the radiographic image capturing system shown in FIG. 1;
FIG. 4 is a fragmentary side elevational view of the mammographic apparatus shown in FIG. 3;
FIG. 5 is a detailed block diagram of the radiographic image capturing system shown in FIG. 1;
FIG. 6 is a detailed block diagram of an image generating apparatus shown in FIG. 5;
FIG. 7 is a flowchart of an operation sequence of the radiographic image capturing system shown in FIG. 1;
FIG. 8 is a flowchart of an operation sequence of an image display apparatus shown in FIG. 1;
FIG. 9A is a view showing an example of setting each image area in a right-eye image;
FIG. 9B is a view showing an example of setting each image area in a left-eye image;
FIGS. 10A through 10C are views showing a screen transition when a planar image is displayed in a 3D image area;
FIGS. 11A and 11B are views showing modified examples of setting each image area in a right-eye image (left-eye image);
FIGS. 12A and 12B are views showing a first modification of a screen transition when a planar image is displayed in a 3D image area; and
FIGS. 13A and 13B are views showing a second modification of a screen transition when a planar image is displayed in a 3D image area.

### DESCRIPTION OF THE PREFERRED EMBODIMENTS

A medical image generating method according to a preferred embodiment of the present invention, in relation to a medical image generating apparatus for carrying out the medical image generating method, will be described in detail below with reference to the accompanying drawings.

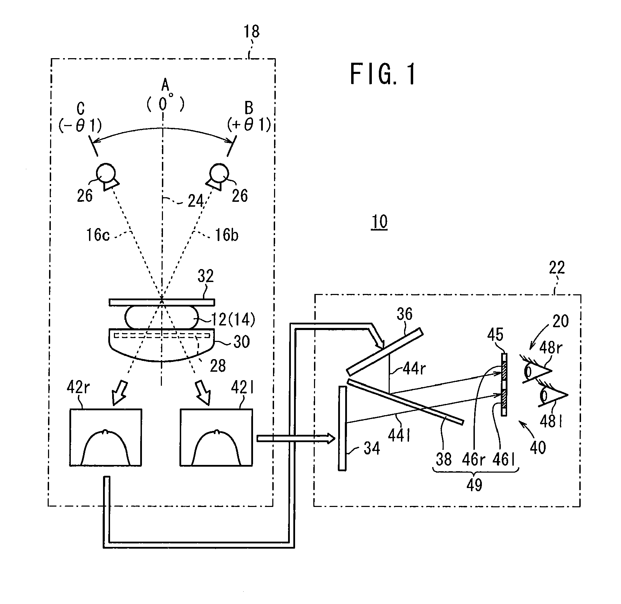

As shown schematically in FIG. 1, a radiographic image capturing system 10 basically comprises a mammographic apparatus 18 (radiographic image capturing apparatus) for performing a stereographic image capturing process by applying radiation 16b, 16c at two different angles to a breast 14 of a subject 12 in order to acquire two radiographic images each covering the breast 14, and an image display apparatus 22 (medical image display apparatus) for enabling an observer 20, such as a doctor, a radiological technician or the like, to view a three-dimensional image (stereographic image) based on two radiographic images obtained from the mammographic apparatus 18. One of the two radiographic images is used as a two-dimensional image (right-eye disparity image) 42r for the right eye 48r of the observer 20, and the other of the two radiographic images is used as a two-dimensional image (left-eye disparity image) 421 for the left eye 481 of the observer 20.

The mammographic apparatus 18 comprises a radiation source 26 for applying radiation 16b, 16c to the breast 14 respectively from a position B, which is angularly spaced an angle of +θ1 from a position A (θ = 0°) on a central axis 24 extending substantially centrally through the breast 14, and from a position C, which is angularly spaced an angle of -θ1 from the position A. The mammographic apparatus 18 further comprises a solid-state detector 28 (radiation detector) for detecting radiation 16b, 16c that has passed through the breast 14, and converting the detected radiation 16b, 16c into radiographic images as a right-eye disparity image 42r and a left-eye disparity image 421, an image capturing base 30 for holding the breast 14 thereon, the image capturing base 30 housing therein the solid-state detector 28, and a compression plate 32 displaceable toward the image capturing base 30 and away from the radiation source 26 for compressing and holding the breast 14 against the image capturing base 30.

In FIG. 1, radiation 16b is applied from the radiation source 26 disposed in position B to the breast 14, and radiation 16b that has passed through the breast 14 is detected and converted into the right-eye disparity image 42r by the solid-state detector 28. Radiation 16c is applied from the radiation source 26 disposed in position C to the breast 14, and radiation 16c that has passed through the breast 14 is detected and converted into the left-eye disparity image 421 by the solid-state detector 28. The central axis 24 extends substantially centrally through the breast 14 and substantially perpendicularly to the upper surface of the image capturing base 30 and the solid-state detector 28.

The image display apparatus 22 comprises a left-eye image display unit 34 for displaying the left-eye disparity image 421, and a right-eye image display unit 36 for displaying the right-eye disparity image 42r. The right-eye image display unit 36 is angularly spaced a predetermined angle from the left-eye image display unit 34. The image display apparatus 22 further comprises a half-silvered mirror 38 disposed in a predetermined position between the left-eye image display unit 34 and the right-eye image display unit 36, for transmitting display light 441 from the left-eye disparity image 421 displayed by the left-eye image display unit 34 therethrough to the observer 20, and for reflecting display light 44r from the right-eye disparity image 42r displayed by the right-eye image display unit 36 therefrom to the observer 20. The image display apparatus 22 also includes a stereoscope 40 to be worn by the observer 20.

The stereoscope 40 comprises a frame 45, a polarizer lens 46r fixed to the frame 45 for the right eye 48r, and a polarizer lens 461 fixed to the frame 45 for the left eye 481. The half-silvered mirror 38, the polarizer lens 46r, and the polarizer lens 461 jointly function as an optical adjuster 49 for adjusting optical paths and polarized states of the display light 44r and the display light 441 (light images).

Through the polarizer lens 46r, the observer 20 sees the right-eye disparity image 42r represented by the display light 44r from the half-silvered mirror 38 with the right eye 48r, and also sees, through the polarizer lens 461, the left-eye disparity image 421 represented by the display light 441 from the half-silvered mirror 38 with the left eye 481, thereby visually recognizing a combined image of the right-eye disparity image 42r and the left-eye disparity image 421 as a stereographic image (three-dimensional image, 3D image).

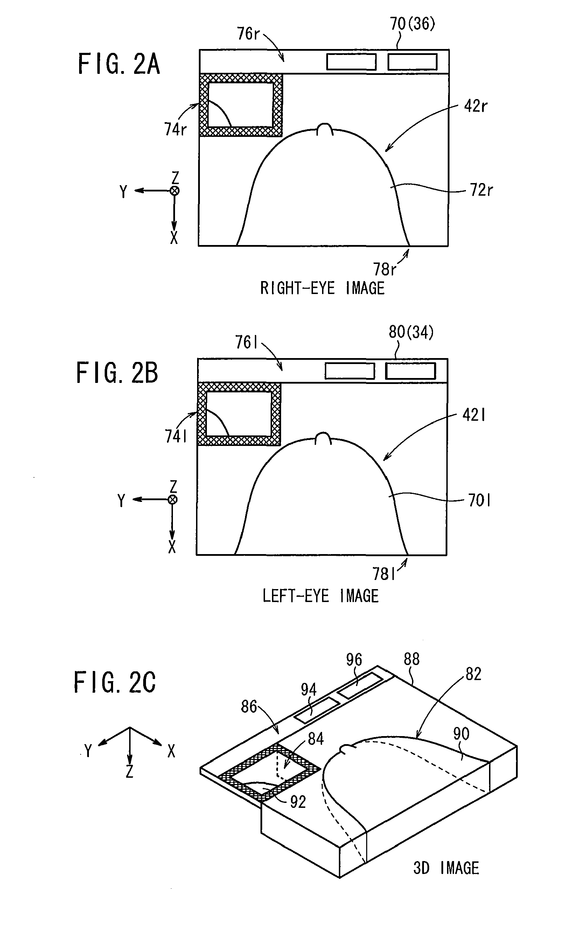

As shown in FIG. 2A, the image display apparatus 22 displays, on a display screen 70 of the right-eye image display unit 36, a right-eye image 78r, which comprises the right-eye disparity image 42r including a breast image 72r representative of the breast 14 therein, a planar image 74r (one image) representative of a portion of the breast 14, and an operation image 76r used for entering operating instructions from the observer 20.

As shown in FIG. 2B, the image display apparatus 22 displays, on a display screen 80 of the left-eye image display unit 34, a left-eye image 781, which comprises the left-eye disparity image 421 including a breast image 721 representative of the breast 14 therein, a planar image 741 representative of a portion of the breast 14, and an operation image 761 used for entering operating instructions from the observer 20. If the right-eye image display unit 36 is the same as the left-eye image display unit 34, then the planar image 74r and the planar image 741 are identical to each other. The planar images 74r, 741 may also simply be referred to as a "planar image 74," free of the alphabetical suffixes "r" and "1".

The observer 20 visually recognizes a combination of the right-eye image 78r and the left-eye image 781 as a virtual display image 88. As shown in FIG. 2C, the virtual display image 88 comprises a stereographic image 82, a planar image 84, and an operation image 86 displayed as a planar representation.

The stereographic image 82 represents a breast image 90 displayed as a stereographic representation of the breast 14. The planar image 84 represents a breast image 92 displayed as a planar representation of a portion of the breast 14. The operation image 86 displays two control buttons 94, 96, which can be operated by the observer 20.

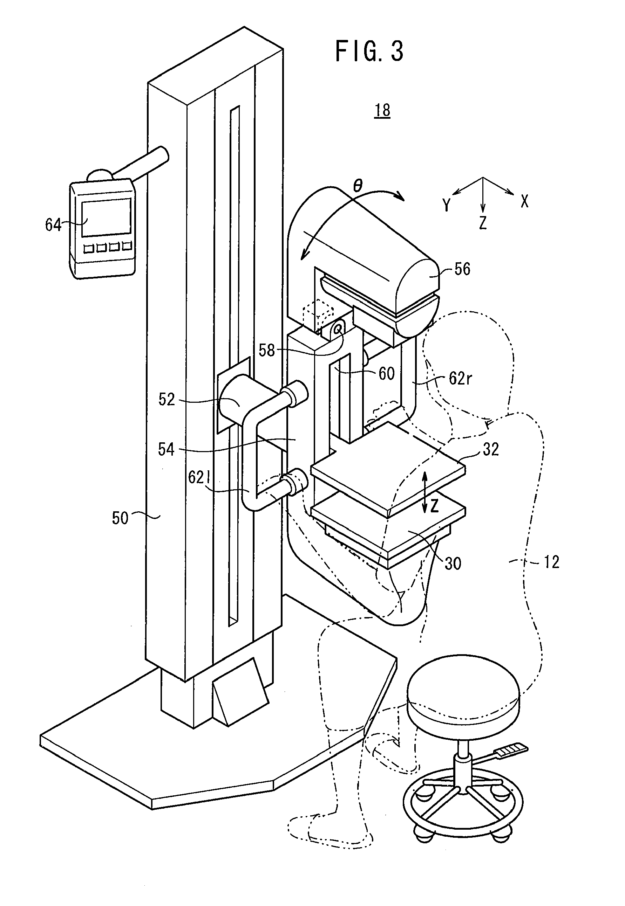

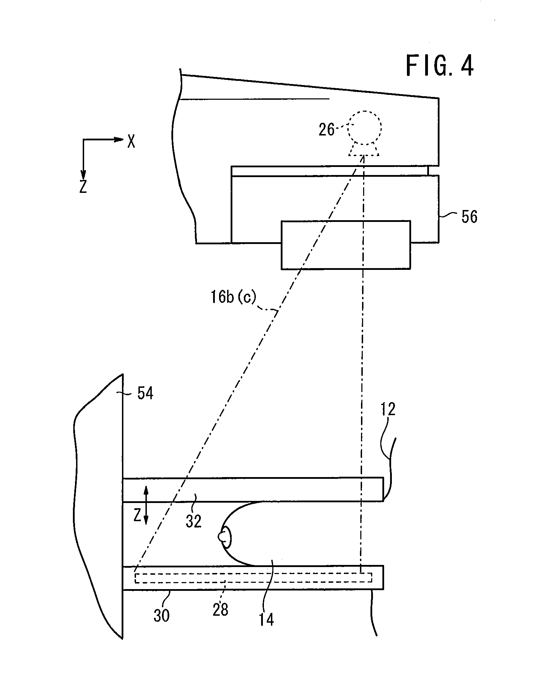

Specific structural details of the mammographic apparatus 18 will be described below with reference to FIGS. 3 and 4.

The mammographic apparatus 18 basically includes an upstanding base 50, a vertical arm 54 fixed to the distal end of a horizontal swing shaft 52 disposed substantially centrally on the base 50, a radiation source housing unit 56 housing therein the radiation source 26 and fixed to an upper end of the arm 54, the image capturing base 30 housing the solid-state detector 28 and fixed to a lower end of the arm 54, and the compression plate 32, which is disposed between the radiation source housing unit 56 and the image capturing base 30. A display control panel 64 is connected to the base 50 for displaying image capturing information including an image capturing region of the subject 12, ID information of the subject 12, etc., and enabling setting of such items of information, as necessary.

When the arm 54, to which the radiation source housing unit 56 and the image capturing base 30 are secured, is angularly moved about the swing shaft 52 in the directions indicated by the arrow θ, the orientation of the radiation source 26 with respect to the breast 14 of the subject 12 is adjusted. The radiation source housing unit 56 is coupled to the arm 54 by a hinge 58, and is angularly movable in the directions indicated by the arrow θ independently of the image capturing base 30.

The arm 54 has a vertical groove 60 defined in a front surface thereof, which faces the subject 12, and extending in the Z-axis directions as indicated by the arrow Z. Handles 62r, 621 to be gripped by the subject 12 are mounted respectively on opposite sides of the arm 54, which are spaced along the Y-axis directions indicated by the arrow Y. The compression plate 32 has a proximal end inserted into the groove 60 and fitted to a mount, not shown. The compression plate 32 is disposed between the radiation source housing unit 56 and the image capturing base 30. The compression plate 32 is vertically displaceable along the arm 54 in the Z-axis directions in unison with the mount.

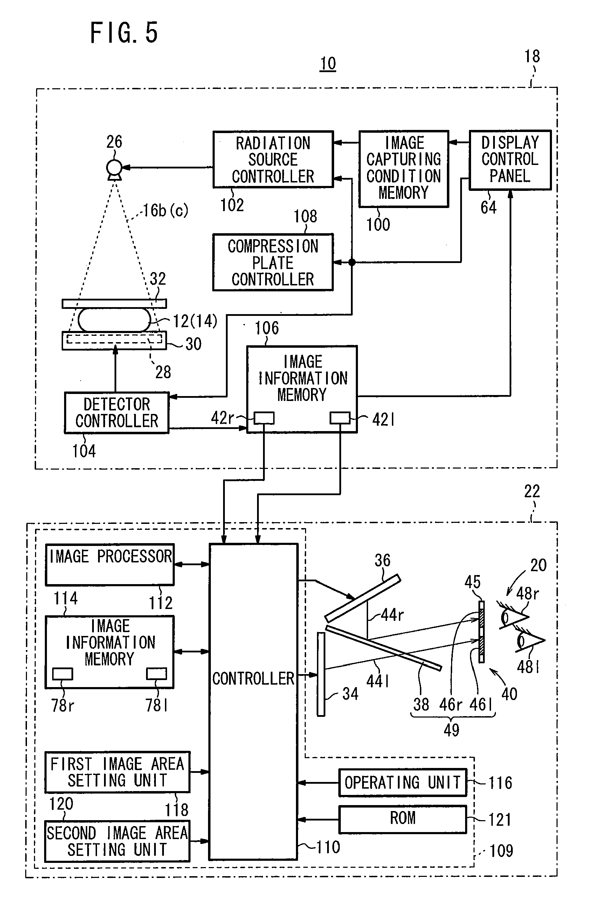

FIG. 5 is a detailed block diagram of the radiographic image capturing system 10.

As shown in FIG. 5, the mammographic apparatus 18 includes, in addition to the components described above with reference to FIGS. 1 through 4, an image capturing condition memory 100, a radiation source controller 102, a detector controller 104, an image information memory 106, and a compression plate controller 108.

The image capturing condition memory 100 stores image capturing conditions including a tube current and a tube voltage of the radiation source 26, irradiation doses and irradiation times for the radiation 16b, 16c, image capturing angles (+θ1, -θ1) and an image capturing sequence for a stereographic image capturing process, three-dimensional positions of the radiation source 26 (the distance between the radiation source 26 and the solid-state detector 28) at the image capturing angles, etc. The image capturing conditions may be set (stored) in the image capturing condition memory 100 when the doctor or radiological technician, i.e., the observer 20, operates the display control panel 64. The radiation source controller 102 energizes the radiation source 26 according to the image capturing conditions. The compression plate controller 108 displaces the compression plate 32 in directions along the Z-axis. The detector controller 104 controls the solid-state detector 28 to store in the image information memory 106 the right-eye disparity image 42r and the left-eye disparity image 421, which have been converted respectively from the radiation 16b, 16c by the solid-state detector 28.

The image display apparatus 22 also includes an image generating apparatus 109 that serves as a medical image generating apparatus (or a medical image generator). The image generating apparatus 109 includes a controller (display controller) 110, an image processor 112, an image information memory 114, an operating unit 116, a first image area setting unit 118, a second image area setting unit 120, and a ROM 121 (recording medium).

The controller 110 acquires the right-eye disparity image 42r and the left-eye disparity image 421, which are stored in the image information memory 106, and controls the image processor 112 to perform image processing on the right-eye disparity image 42r and the left-eye disparity image 421. The controller 110 displays the processed right-eye image 78r on the display screen 70 (see FIG. 2A) of the right-eye image display unit 36, and also displays the processed left-eye image 781 on the display screen 80 (see FIG. 2B) of the left-eye image display unit 34. The controller 110 can also perform other processing sequences according to operating instructions input from the observer 20 through the operating unit 116.

The image information memory 114 stores the processed right-eye image 78r and the processed left-eye image 781.

The first image area setting unit 118 sets 3D image areas (image areas to be displayed as a stereographic representation) from within the display areas of the right-eye image 78r and the left-eye image 781. The second image area setting unit 120 sets 2D image areas (image areas to be displayed as a planar representation) from within the respective 3D image areas.

The ROM 121 stores a program to be executed by a computer of the image generating apparatus 109 or the like on the right-eye disparity image 42r and the left-eye disparity image 421, so as to be presented for three-dimensional image display based on binocular disparity.

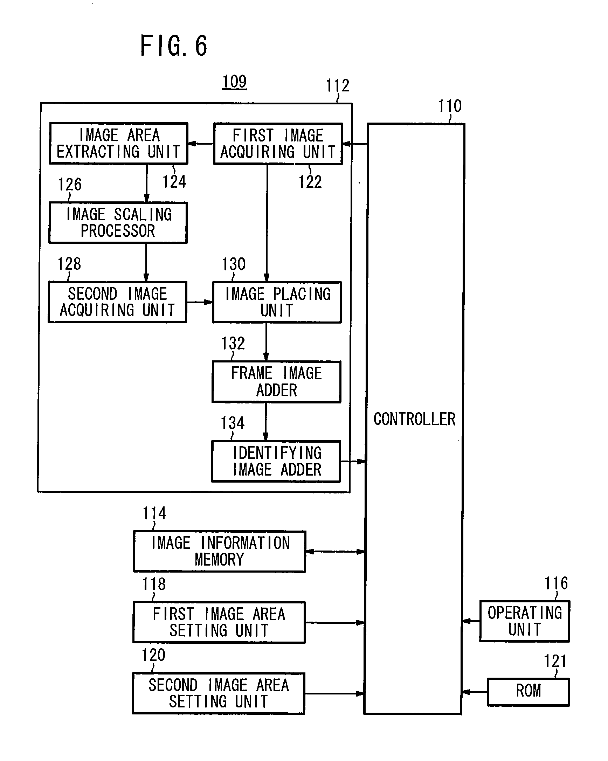

FIG. 6 is a detailed block diagram of the image generating apparatus 109 shown in FIG. 5.

The image generating apparatus 109 includes a first image acquiring unit 122 for acquiring the right-eye disparity image 42r and the left-eye disparity image 421 (a pair of disparity images) from the mammographic apparatus 18, an image area extracting unit 124 for extracting a prescribed image area (hereinafter referred to as an "extracted area") from a 3D image area, an image scaling processor 126 for scaling a planar image, which corresponds to the position of the extracted area extracted by the image area extracting unit 124, a second image acquiring unit 128 for acquiring processed planar images 74 that have been scaled by the image scaling processor 126, an image placing unit 130 for placing the right-eye disparity image 42r, the left-eye disparity image 421, and the processed planar images in prescribed positions, a frame image adder 132 for adding an image of a light blocking frame having a low brightness level to a boundary between the 3D image area and the 2D image area, and an identifying image adder 134 (first identifying image adder, second identifying image adder) for adding an image of an identification mark (e.g., an indication "R" or "L") for identifying either one of the disparity images the planar image 74 of which has been acquired by the second image acquiring unit 128, and an image (e.g., a solid-line frame or the like) of an identification mark for identifying the extracted image.

The image scaling processor 126 performs a scaling process for matching the size of the extracted area to the size of the 2D image area, for example, depending on a determined image magnification ratio.

The image placing unit 130 places the processed planar images in the 2D image areas of the right-eye image 78r and the left-eye image 781. The image placing unit 130 also places the right-eye disparity image 42r and the left-eye disparity image 421, respectively, in the 3D image areas (apart from areas overlapping with the 2D image areas) for the right-eye image 78r and the left-eye image 781.

The radiographic image capturing system 10, which includes the mammographic apparatus 18 and the image display apparatus 22 according to the present embodiment, is constructed as described above.

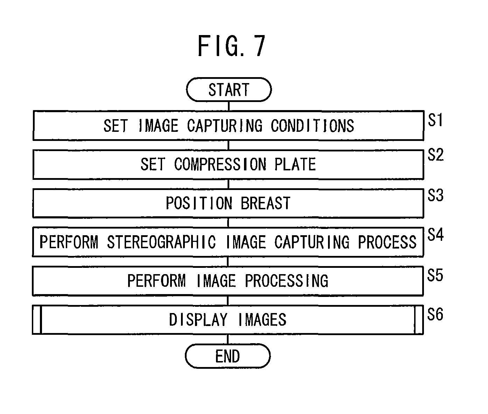

Operations of the radiographic image capturing system 10, i.e., an image display method carried out by the image display apparatus 22, will be described below with reference to the flowchart shown in FIG. 7.

In step S1 shown in FIG. 7, prior to performing a stereographic image capturing process on the breast 14 (see FIG. 5), the doctor or radiological technician, i.e., the observer 20, operates the display control panel 64 of the mammographic apparatus 18 to store image capturing conditions depending on the breast 14 of the subject 12 (see FIGS. 1 through 5) in the image capturing condition memory 100.

In step S2, as shown in FIG. 3, the doctor or radiological technician inserts the proximal end of the compression plate 32 into the groove 60 and fits the proximal end of the compression plate 32 to the mount, thereby placing the compression plate 32 between the radiation source housing unit 56 and the image capturing base 30.

In step S3, the doctor or radiological technician positions the breast 14 of the subject 12. More specifically, the doctor or radiological technician places the breast 14 on the image capturing base 30 at a position confronting the compression plate 32, and operates the display control panel 64 to energize the compression plate controller 108 to move the compression plate 32 toward the image capturing base 30, in a downward direction as indicated by the arrow Z (see FIG. 5). The breast 14 is compressed and secured in place by the image capturing base 30 and the compression plate 32.

After the preparatory process of steps S1 through S3 has been completed, the mammographic apparatus 18 energizes the radiation source 26 to perform a stereographic image capturing process on the breast 14 in step S4.

More specifically, when the doctor or radiological technician operates the display control panel 64 to instruct the mammographic apparatus 18 to start a stereographic image capturing process, the radiation source controller 102 (see FIG. 5) turns the radiation source housing unit 56 about the hinge 58 to place the radiation source 26 in position B. Then, the doctor or radiological technician turns on an exposure switch, not shown, displayed on the display control panel 64, thereby causing the radiation source controller 102 to control the radiation source 26 in position B according to the image capturing conditions stored in the image capturing condition memory 100 (see FIG. 1).

The radiation source 26 in position B emits radiation 16b, which is applied through the compression plate 32 to the breast 14. Radiation 16b is transmitted through the breast 14, and is detected by the solid-state detector 28 as representing a first radiographic image (right-eye disparity image 42r). The detector controller 104 controls the solid-state detector 28 to acquire the first radiographic image (right-eye disparity image 42r) and stores the acquired right-eye disparity image 42r in the image information memory 106.

Upon completion of capturing the right-eye disparity image 42r at position B, the mammographic apparatus 18 moves the radiation source 26 to position C, and captures a second radiographic image at position C in the same manner as having captured the first radiographic image at position B.

More specifically, when completion of capturing the right-eye disparity image 42r is displayed on the display control panel 64, the doctor or radiological technician operates the display control panel 64 to instruct the mammographic apparatus 18 to start capturing a second radiographic image.

The radiation source controller 102 turns the radiation source housing unit 56 about the hinge 58, so as to move the radiation source 26 from position B to position C, and places the radiation source 26 in position C. Then, the doctor or radiological technician turns on the exposure switch, thereby causing the radiation source controller 102 to control the radiation source 26 in position C according to the image capturing conditions stored in the image capturing condition memory 100.

The radiation source 26 in position C emits radiation 16c, which is applied through the compression plate 32 to the breast 14. Radiation 16c is transmitted through the breast 14, and is detected by the solid-state detector 28 as representing a second radiographic image (left-eye disparity image 421). The detector controller 104 controls the solid-state detector 28 to acquire the second radiographic image (left-eye disparity image 421) and stores the acquired left-eye disparity image 421 in the image information memory 106.

Upon completion of the stereographic image capturing process in step S4, the image information memory 106 stores therein two radiographic images, i.e., the right-eye disparity image 42r and the left-eye disparity image 421, each of which comprises a two-dimensional image.

Thereafter, in step S5, an image processor (not shown) of the mammographic apparatus 18 (see FIGS. 1, 4 and 5) automatically acquires the right-eye disparity image 42r and the left-eye disparity image 421 from the image information memory 106, or acquires the right-eye disparity image 42r and the left-eye disparity image 421 based on an image acquiring instruction input from the observer 20 through the operating unit 116. The image processor then performs predetermined image processing, including frequency emphasis, density gradation correction, etc., on the right-eye disparity image 42r and the left-eye disparity image 421. The image processor then stores the right-eye disparity image 42r and the left-eye disparity image 421, which have been processed, in the image information memory 106.

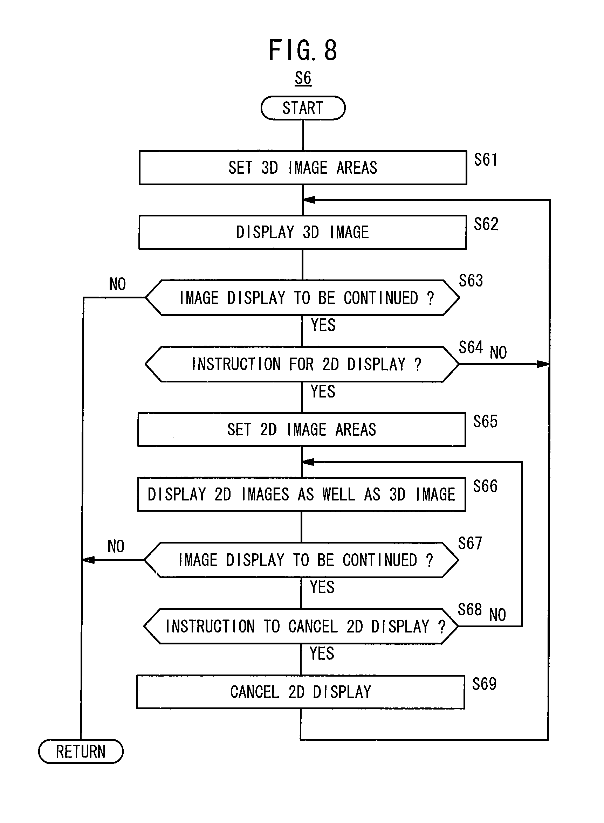

In step S6, the controller 110 controls the image display apparatus 22 to display appropriate diagnostic images. Operations of the image display apparatus 22 to display such diagnostic images will be described below with reference to the flowchart shown in FIG. 8.

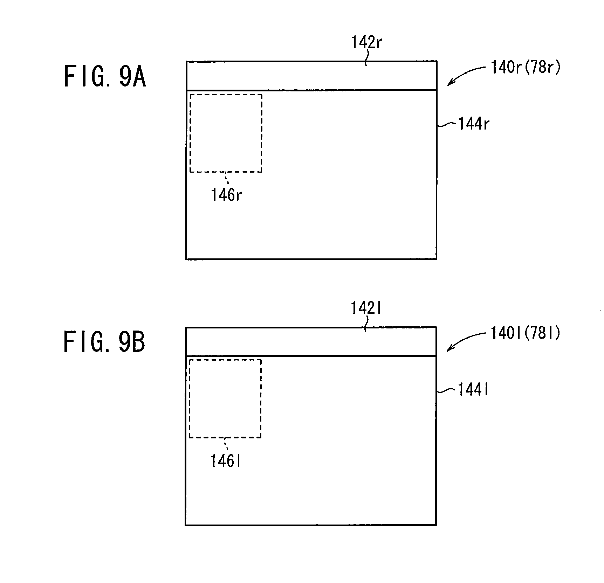

In step S61 shown in FIG. 8, the first image area setting unit 118 sets 3D image areas. More specifically, as shown in FIG. 9A, the first image area setting unit 118 sets as a 3D image area 144r a rectangular image area, apart from an operation image area 142r, in a display area 140r for the right-eye image 78r. As shown in FIG. 9B, the first image area setting unit 118 also sets as a 3D image area 1441 a rectangular image area, apart from an operation image area 1421, in a display area 1401 for the left-eye image 781. Positions and sizes of such 3D image areas may be given according to predetermined values, or may be varied freely by the observer 20 through the operating unit 116.

In step S62, the controller 110 controls the right-eye image display unit 36 and the left-eye image display unit 34 to display a 3D image. More specifically, the first image acquiring unit 122 (see FIG. 6) acquires the right-eye disparity image 42r and the left-eye disparity image 421 from the controller 110, and supplies the disparity images as a pair to the image placing unit 130. The image placing unit 130 places the right-eye disparity image 42r in the 3D image area 144r (see FIG. 9A), thereby generating a right-eye image 78r. Similarly, the image placing unit 130 places the left-eye disparity image 421 in the 3D image area 1441 (see FIG. 9B), thereby generating a left-eye image 781. For appropriately placing images in the 3D image areas, the image placing unit 130 may carry out known image processing processes, including trimming, image scaling, etc., on the right-eye disparity image 42r and the left-eye disparity image 421.

The image placing unit 130 then supplies the right-eye image 78r and the left-eye image 781, which have been generated via the frame image adder 132 and the identifying image adder 134, to the controller 110. At this time, the frame image adder 132 and the identifying image adder 134 do not perform any image processing.

As shown in FIG. 5, the controller 110 displays the right-eye image 78r on the display screen 70 of the right-eye image display unit 36, and displays the left-eye image 781 on the display screen 80 of the left-eye image display unit 34. Display light 44r from the right-eye image 78r, which is displayed on the display screen 70 of the right-eye image display unit 36, travels downward toward the half-silvered mirror 38, whereas display light 441 from the left-eye image 781, which is displayed on the display screen 80 of the left-eye image display unit 34, travels obliquely upward toward the half-silvered mirror 38.

The half-silvered mirror 38 reflects therefrom the display light 44r toward the stereoscope 40, and transmits therethrough the display light 44r toward the stereoscope 40, which is worn by the observer 20. The polarizer lens 46r of the stereoscope 40 transmits therethrough only display light 44r that is parallel to the absorption axis of the polarizer lens 46r, while the polarizer lens 461 of the stereoscope 40 transmits therethrough only display light 441 that is parallel to the absorption axis of the polarizer lens 461. The observer 20 who wears the stereoscope 40 sees the right-eye image 78r represented by the display light 44r with the right eye 48r, and sees the left-eye image 781 represented by the display light 441 with the left eye 481, thereby visually recognizing a stereographic image 82 (see FIG. 2C) of the breast 14.

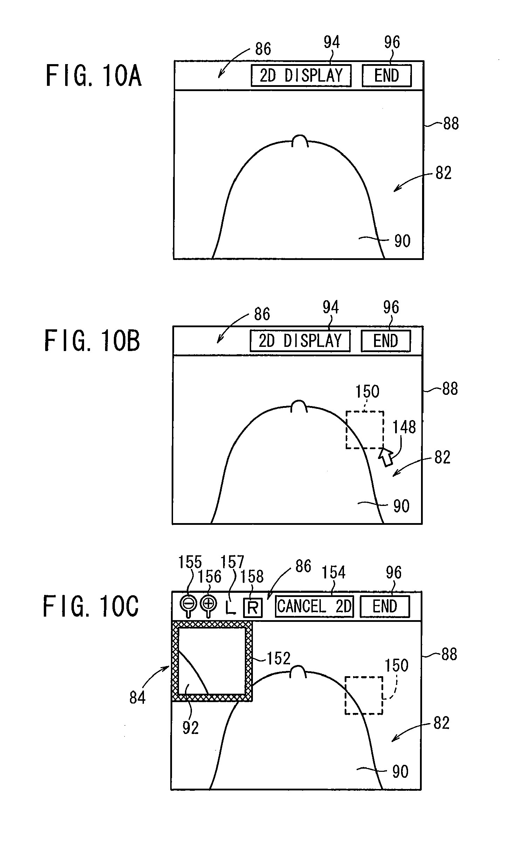

In step S63, the controller 110 determines whether the above image displaying process should be continued, specifically based on whether an "END" button 96 shown in FIG. 10A has been clicked or not. If it is judged that the "END" button 96 has been clicked in step S63, then the image displaying process is brought to an end. If it is judged that the "END" button 96 has not been clicked in step S63, then control proceeds from step S63 to step S64.

In step S64, the controller 110 determines whether there is an instruction for starting a 2D display, specifically based on whether a "2D DISPLAY" button 94 shown in FIG. 10A has been clicked or not. If it is judged that the "2D DISPLAY" button 94 has not been clicked, then control goes back to step S62. Thereafter, steps S62 through S64 are looped through again until the "2D DISPLAY" button 94 is clicked. If it is judged that the "2D DISPLAY" button 94 has been clicked in step S64, then control proceeds from step S64 to step S65.

In step S65, the second image area setting unit 120 sets 2D image areas. More specifically, as shown in FIG. 9A, the second image area setting unit 120 sets as a 2D image area 146r a rectangular image area, indicated by the broken lines, in the 3D image area 144r for the right-eye image 78r. As shown in FIG. 9B, the second image area setting unit 120 also sets as a 2D image area 1461 a rectangular image area, indicated by the broken lines, in the 3D image area 1441 for the left-eye image 781. Positions and sizes of such 2D image areas may be given according to predetermined values, or may be varied freely by the observer 20 through the operating unit 116.

In step S66, the controller 110 controls the right-eye image display unit 36 and the left-eye image display unit 34 to display 2D images together with the 3D image.

More specifically, the first image acquiring unit 122 acquires the right-eye disparity image 42r and the left-eye disparity image 421 from the controller 110, and supplies the disparity images as a pair to the image placing unit 130.

The image area extracting unit 124 extracts image data of a certain image area (extracted area) in either one of the right-eye disparity image 42r or the left-eye disparity image 421 supplied from the first image acquiring unit 122.

More specifically, as shown in FIG. 10B, the doctor or radiological technician indicates an area of interest with a pointer 148 using the operating unit 116, such as a mouse, for example, and drags the area of interest. Then, an area extracting frame 150, indicated by the broken lines, is added to the virtual display image 88. The doctor or radiological technician clicks the "2D DISPLAY" button 94, thereby determining an image area enclosed by the area extracting frame 150 as an extracted area.

The image scaling processor 126 shown in FIG. 6 scales the image data in order to match the size of the extracted area to the size of the 2D image areas 146r, 1461 (see FIGS. 9A and 9B). The image scaling processor 126 may scale the image data according to a known interpolation process, or an arithmetic algorithm depending on a given image magnification ratio.

The second image acquiring unit 128 acquires the image data supplied from the image scaling processor 126 as planar images 74r, 741. The second image acquiring unit 128 may acquire either one of the right-eye disparity image 42r or the left-eye disparity image 421 as the planar images 74r, 741. Alternatively, if the mammographic apparatus 18 acquired a radiographic image in a frontal image capturing process, in which the radiation source 26 was placed in position A in FIG. 1, then the second image acquiring unit 128 may use the acquired frontal radiographic image as the planar images 74r, 741.

The image placing unit 130 acquires the right-eye disparity image 42r and the left-eye disparity image 421 from the first image acquiring unit 122, and also acquires the planar images 74r, 741 from the second image acquiring unit 128. The image placing unit 130 then places the planar image 74r in the 2D image area 146r for the right-eye image 78r, places the right-eye disparity image 42r in the 3D image area 144r (apart from an area that overlaps with the 2D image area 146r) for the right-eye image 78r, and places the operation image 76r in the operation image area 142r for the right-eye image 78r, thereby generating a right-eye image 78r. The image placing unit 130 also places the planar image 741 in the 2D image area 1461 for the left-eye image 781, places the left-eye disparity image 421 in the 3D image area 1441 (apart from an area that overlaps with the 2D image area 1461) for the left-eye image 781, and places the operation image 761 in the operation image area 1421 for the left-eye image 781, thereby generating a left-eye image 781.

The frame image adder 132 acquires the right-eye image 78r and the left-eye image 781 from the image placing unit 130. The frame image adder 132 adds light blocking frames 152 (shown in hatching in FIG. 10C) having a low brightness level in the right-eye image display unit 36 and the left-eye image display unit 34, near boundaries between the 3D image areas 144r, 1441 and the 2D image areas 146r, 1461 for the paired two-dimensional images. The light blocking frames 152 are effective to reduce glare from the planar images 74 when the observer 20 observes the planar images 74, so that the observer 20 can observe the planar images 74 more easily. Since the light blocking frames 152 clearly separate the stereographic image 82 and the planar image 74 (see FIG. 2C) from each other at a boundary thereof, the observer 20 finds it easy to observe the stereographic image 82 and the planar image 74 independently of each other.

The identifying image adder 134 acquires the right-eye image 78r and the left-eye image 781 from the frame image adder 132. The identifying image adder 134 adds the area extracting frame 150, which defines an extracted area, to the paired two-dimensional images, thereby making the relationship between the displayed positions of the stereographic image 82 and the planar image 74 capable of being visualized, thus making it easy for the observer 20 to recognize the relationship.

The controller 110 acquires the right-eye image 78r and the left-eye image 781, which have been processed, from the identifying image adder 134. Similar to step S62, the controller 110 displays the right-eye image 78r on the display screen 70 of the right-eye image display unit 36, and displays the left-eye image 781 on the display screen 80 of the left-eye image display unit 34, thereby producing a virtual display image 88 as shown in FIG. 10C.

When the "2D DISPLAY" button 94 is clicked, icons 155, 156, 157, 158 are newly displayed on the left side of the operation image 86.

When the icon 155 is clicked, the image magnification ratio is reduced from the present value, thereby displaying the planar images 74r, 741 at a reduced scale, respectively, in the 2D image areas 146r, 1461 (see FIGS. 9A and 9B). When the icon 156 is clicked, the image magnification ratio is increased from the present value, thereby displaying the planar images 74r, 741 at an enlarged scale, respectively, in the 2D image areas 146r, 1461 (see FIGS. 9A and 9B).

The icons 157, 158, which are displayed as "L" and "R", respectively, represent the types of planar images 74r, 741 that are currently displayed in the respective 2D image areas 146r, 1461. In FIG. 10C, for example, "R" enclosed by a solid-line frame implies that an image extracted from the right-eye disparity image 42r is displayed as a 2D image. On the other hand, "L" enclosed by a solid-line frame, not shown, implies that an image extracted from the left-eye disparity image 421 is displayed as the planar images 74r, 741.

As shown in FIG. 10C, by clicking the icon 157 that is not enclosed by a solid-line frame, a frame is newly added to "L", whereas the frame added to "R" disappears. In addition, the currently displayed planar image 74 changes from the image extracted from the right-eye disparity image 42r to the image extracted from the left-eye disparity image 421.

Therefore, the observer 20 can easily visually recognize or selectively vary the types of planar images 74r, 741 that are currently displayed.

In step S67 shown in FIG. 8, the controller 110 determines whether the above image displaying process should be continued, specifically, based on whether the "END" button 96 shown in FIG. 10C has been clicked or not. If it is judged that the "END" button 96 has been clicked in step S67, then the image displaying process is brought to an end. If it is judged that the "END" button 96 has not been clicked in step S67, then control proceeds from step S67 to step S68.

In step S68, the controller 110 determines whether there is an instruction for canceling the 2D display, specifically based on whether the "CANCEL 2D DISPLAY" button 154 shown in FIG. 10C has been clicked or not. When step S66 is started, the "2D DISPLAY" button 94 shown in FIG. 10B is replaced with the "CANCEL 2D DISPLAY" button 154 shown in FIG. 10C. If it is judged that the "CANCEL 2D DISPLAY" button 154 has not been clicked in step S68, then control returns to step S66. Thereafter, steps S66 through S68 are looped through again until the "CANCEL 2D DISPLAY" button 154 is clicked. If it is judged that the "CANCEL 2D DISPLAY" button 154 has been clicked in step S68, then control proceeds from step S68 to step S69.

In step S69, the 2D display is canceled. More specifically, the second image area setting unit 120 shown in FIG. 6 sends a signal indicating cancellation of the setting of the 2D image areas 146r, 1461, via the controller 110 to the image processor 112. The second image acquiring unit 128 stops supplying the image data representative of the planar images 74r, 741 to the image placing unit 130. As already described above with reference to step S62, the stereographic image 82 is displayed as the virtual display image 88, and the planar image 84 is not displayed thereover.

Then, control returns to step S62, and steps S62 through S69 are looped through successively until the image displaying process has ended.

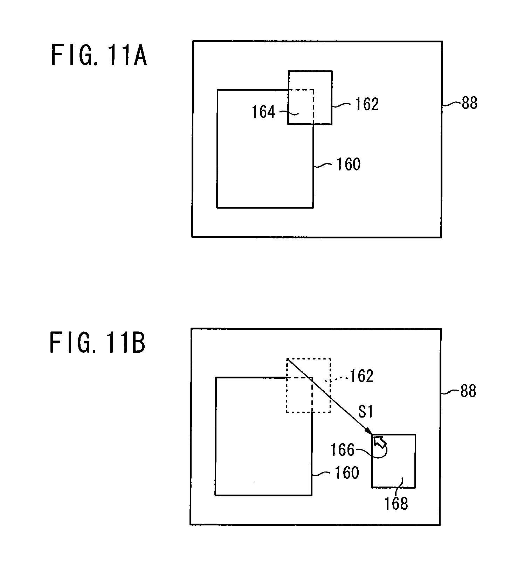

In the present embodiment shown in FIGS. 9A and 9B, the 2D image area 146r (1461) is included within the 3D image area 144r (1441). However, the 2D image area 146r (1461) need not necessarily be included within the 3D image area 144r (1441). According to a modified example, as shown in FIG. 11A, a 3D image area 160, i.e., an image area for displaying an image as a stereographic representation, and a 2D image area 162, i.e., an image area for displaying an image as a planar representation, overlap each other in an overlapping area 164. A portion of the planar image 74r (741) is preferentially displayed in the overlapping area 164.

According to another modified example, as shown in FIG. 11B, the 2D image area 162 can be moved based on an operating instruction entered from the observer 20 via the operating unit 116. For example, a GUI may be employed for moving the 2D image area 162 from a starting point of a pointer 166 (2D image area 162) to an end point (2D image area 168) along the direction indicated by the arrow S1, when the observer 20 operates the operating unit 116 (e.g., drags a mouse).

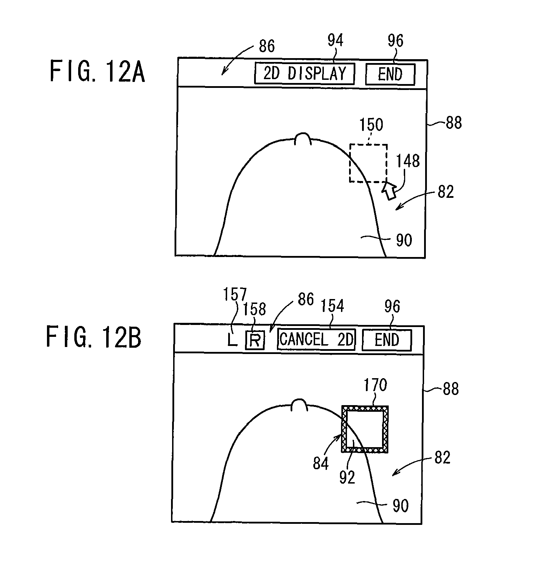

The planar image 84 may be displayed in the image area indicated by the area extracting frame 150, as shown in FIG. 10B. For example, as shown in FIG. 12A, after the area extracting frame 150 has been set to a given position and range using a pointer 148, the observer 20 clicks the "2D DISPLAY" button 94. Then, the area enclosed by the area extracting frame 150 is set as the 2D image areas 146r, 1461 (see FIGS. 9A and 9B) for displaying the planar image 84. A light blocking frame 170 (see FIG. 12B) may be displayed on an outer edge of the area enclosed by the area extracting frame 150. In this case, the image magnification ratio of the planer image 84 is an equal-size ratio (100%).

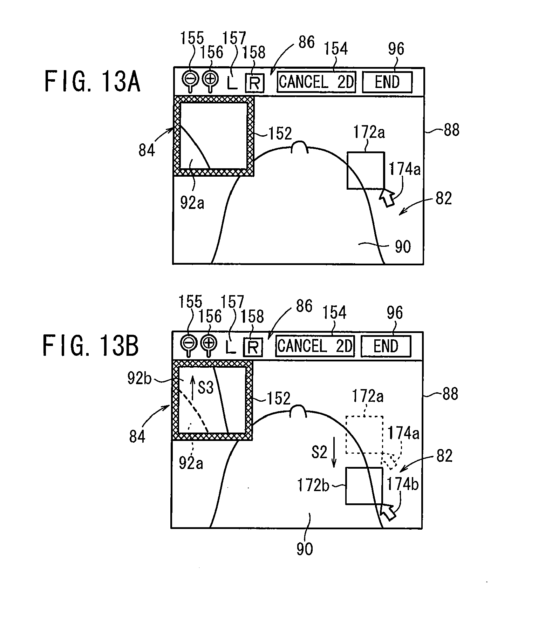

The area extracting frame 150 shown in FIG. 10C may be varied in position, and the planar image 84 may be immediately displayed depending on the varied position of the area extracting frame 150. For example, as shown in FIG. 13A, a planar image 84 (breast image 92a) extracted by an area extracting frame 172a is displayed. The observer 20 drags a mouse, not shown, in order to move the image from a starting point indicated by a pointer 174a along the direction indicated by the arrow S2 (see FIG. 13B), whereupon the area extracting frame 172a also is moved along the direction indicated by the arrow S2.

As shown in FIG. 13B, the planar image 84 is immediately updated such that the planar image 84 is continuously shifted along the direction indicated by the arrow S3, which is opposite to the direction indicated by the arrow S2. When the observer 20 stops dragging the mouse, a planar image 84 (breast image 92b) extracted by an area extracting frame 172b at a position indicated by a pointer 174b is displayed.

According to the present invention, as described above, the 3D image areas 144r, 1441 are set respectively from within the display areas 140r, 1401 of the right-eye image display unit 36 and the left-eye image display unit 34, and the 2D image areas 146r, 1461 are set respectively from within the 3D image areas 144r, 1441. The right-eye disparity image 42r and the left-eye disparity image 421, which are to be displayed respectively in the 3D image areas 144r, 1441, and the planar images 74, which are to be displayed respectively in the 2D image areas 146r, 1461, are acquired. The planar images 74 are placed respectively in the 2D image areas 146r, 1461, and the right-eye disparity image 42r and the left-eye disparity image 421 are placed in the 3D image areas 144r, 1441, apart from the 2D image areas 146r, 1461, thereby generating the right-eye image 78r and the left-eye image 781. Consequently, the planar images are placed over the stereographic image for visual observation, and hence the display space required for the image areas is reduced. Since the amount of information of the diagnostic images that can be observed at a time is increased, the image diagnostic ability is greatly increased.

## Claims

1. A medical image generating apparatus (109) for generating a pair of two-dimensional images (741, 74r) to be displayed respectively on a pair of display units (34, 36) for providing a stereographic image display based on binocular disparity, comprising:
a first image area setting unit (118) for setting image areas (1441, 144r, 160) for stereographic image display respectively from within display areas (1401, 140r) of the respective display units (34, 36);
a second image area setting unit (120) for setting image areas (1461, 146r, 162) for planar image display from within the image areas (1441, 144r, 160) for stereographic image display, set by the first image area setting unit (118);
a first image acquiring unit (122) for acquiring a pair of disparity images (421, 42r) to be displayed respectively in the image areas (1441, 144r, 160) for stereographic image display, set by the first image area setting unit (118);
a second image acquiring unit (128) for acquiring one image (74) to be displayed in the image areas (1461, 146r, 162) for planar image display, set by the second image area setting unit (120); and
an image placing unit (130) for placing the one image (74) acquired by the second image acquiring unit (128) in the image areas (1461, 146r, 162) for planar image display, and placing the disparity images (421, 42r) acquired by the first image acquiring unit (122) in the image areas (1441, 144r, 160) for stereographic image display, apart from the image areas (1461, 146r, 162) for planar image display, thereby generating the pair of two-dimensional images (741, 74r).

2. The medical image generating apparatus (109) according to claim 1, further comprising:
a frame image adder (132) for adding an image (152, 170) of a light blocking frame, near a boundary between the image areas (1441, 144r, 160) for stereographic image display and the image areas (1461, 146r, 162) for planar image display.

3. The medical image generating apparatus (109) according to claim 1, further comprising:
a first identifying image adder (134) for adding an image (157, 158) of an identification mark for identifying either one of the disparity images (421, 42r), one image (74) of which has been acquired by the second image acquiring unit (128).

4. The medical image generating apparatus (109) according to claim 1, further comprising:
an image area extracting unit (124) for extracting a prescribed image area from within the image areas (1441, 144r, 160) for stereographic image display,
wherein the second image acquiring unit (128) acquires, as the one image (74), a planar image (74) corresponding to a position of the prescribed image area extracted by the image area extracting unit (124).

5. The medical image generating apparatus (109) according to claim 4, further comprising:
an image scaling processor (126) for scaling the planar image (74) to match a size of the prescribed image area to a size of the image areas (1461, 146r, 162) for planar image display.

6. The medical image generating apparatus (109) according to claim 5, wherein the image scaling processor (126) further scales the planar image (74) displayed in the image areas (1461, 146r, 162) for planar image display, depending on an image magnification ratio that has been set.

7. The medical image generating apparatus (109) according to claim 4, further comprising:
an identifying image adder (134) for adding an image (150, 172a, 172b) of an identification mark, which defines the prescribed image area.

8. The medical image generating apparatus (109) according to claim 1, wherein the pair of disparity images (421, 42r) comprises a pair of radiographic images (421, 42r) acquired by a radiation detector (28), which detects radiation (16b, 16c) that has passed through a subject (12), when a radiation source (26) positioned at respective different angles with respect to the radiation detector (28) applies the radiation (16b, 16c) through the subject (12) to the radiation detector (28).

9. A medical image display apparatus (22) comprising:
a pair of display units (34, 36);
a medical image generating apparatus (109) for generating a pair of two-dimensional images (741, 74r) to be displayed respectively on the display units (34, 36) for providing a stereographic image display based on binocular disparity; and
an optical adjuster (49) for adjusting optical paths and polarized states of light images (441, 44r) from the display units (34, 36) for thereby enabling an observer (20) to visually recognize the light images (441, 44r) as a stereographic representation,
wherein the medical image generating apparatus (109) comprises:
a first image area setting unit (118) for setting image areas (1441, 144r, 160) for stereographic image display respectively from within display areas (1401, 140r) of the respective display units (34, 36);
a second image area setting unit (120) for setting image areas (1461, 146r, 162) for planar image display from within the image areas (1441, 144r, 160) for stereographic image display, set by the first image area setting unit (118);
a first image acquiring unit (122) for acquiring a pair of disparity images (421, 42r) to be displayed respectively in the image areas (1441, 144r, 160) for stereographic image display, set by the first image area setting unit (118);
a second image acquiring unit (128) for acquiring one image (74) to be displayed in the image areas (1461, 146r, 162) for planar image display, set by the second image area setting unit (120); and
an image placing unit (130) for placing the one image (74) acquired by the second image acquiring unit (128) in the image areas (1461, 146r, 162) for planar image display, and placing the disparity images (421, 42r) acquired by the first image acquiring unit (122) in the image areas (1441, 144r, 160) for stereographic image display, apart from the image areas (1461, 146r, 162) for planar image display, thereby generating the pair of two-dimensional images (741, 74r).

10. A medical image generating method for generating a pair of two-dimensional images (741, 74r) to be displayed respectively on a pair of display units (34, 36) for providing a stereographic image display based on binocular disparity, comprising the steps of:
setting image areas (1441, 144r, 160) for stereographic image display respectively from within display areas (1401, 140r) of the respective display units (34, 36);
setting image areas (1461, 146r, 162) for planar image display from within the image areas (1441, 144r, 160) for stereographic image display, which have been set;
acquiring a pair of disparity images (421, 42r) to be displayed respectively in the image areas for stereographic image display, which have been set;
acquiring one image (74) to be displayed in the image areas (1461, 146r, 162) for planar image display, which have been set; and
placing the one image (74) that has been acquired, in the image areas (1461, 146r, 162) for planar image display, and placing the disparity images (421, 42r) that have been acquired, in the image areas (1441, 144r, 160) for stereographic image display, apart from the image areas (1461, 146r, 162) for planar image display, thereby generating the pair of two-dimensional images (741, 74r).

11. A recording medium (121) storing a program to be executed by a computer on a pair of disparity images (421, 42r) to be displayed for providing a stereographic image display based on binocular disparity, the program enabling the computer to function as:
a first image area setting unit (118) for setting image areas (1441, 144r, 160) for stereographic image display respectively from within display areas (1401, 140r) of a pair of respective display units (34, 36);
a second image area setting unit (120) for setting image areas (1461, 146r, 162) for planar image display from within the image areas (1441, 144r, 160) for stereographic image display, set by the first image area setting unit (118);
a first image acquiring unit (122) for acquiring the pair of disparity images (421, 42r) to be displayed respectively in the image areas (1441, 144r, 160) for stereographic image display, set by the first image area setting unit (118);
a second image acquiring unit (128) for acquiring one image (74) to be displayed in the image areas (1461, 146r, 162) for planar image display, set by the second image area setting unit (120); and
an image placing unit (130) for placing the one image (74) acquired by the second image acquiring unit (128) in the image areas (1461, 146r, 162) for planar image display, and placing the disparity images (421, 42r) acquired by the first image acquiring unit (122) in the image areas (1441, 144r, 160) for stereographic image display, apart from the image areas (1461, 146r, 162) for planar image display, thereby generating the pair of two-dimensional images (741, 74r).
